# EUROPEAN PATENT APPLICATION

(11) **EP 2 990 975 A1**
(43) Date of publication of application: **02.03.2016**
(21) Application number: 15180792.2
(22) Date of filing: 12.08.2015
(51) Int. Cl.: G06F 19/00, A61M 16/00, A61B 5/00, A61B 5/087

(54) **BREATHABLE GAS SUPPLY SYSTEM, CONTROL METHOD THEREOF AND COMPUTER PROGRAM PRODUCT IMPLEMENTING THE METHOD**

(30) Priority: 28.08.2014 TW 103129633
(71) Applicant: Apex Medical Corp., New Taipei City, 23679 (TW)
(72) Inventor: Lee, Da-Long, 23679 New Taipei City (TW)
(74) Representative: Schwerbrock, Florian

(57) **Abstract**

A breathable gas supply system (10) comprises a flow sensor (12), a control unit (13) and a storage unit (14). The flow sensor (12) detects a gas state of a flow channel (20). The control unit (13) provides an initial template value and continuously receives sampled breath values from the flow sensor (12) to repetitively perform a first breath sampling process so as to generate a first template value for replacing the initial template value; then the control unit (13) continuously receives sampled breath values from the flow sensor (12) to repetitively perform a second breath sampling process so as to generate a second template value; finally the control unit (13) integrates the first template value and the second template value to obtain an updated template value for replacing the first template value, and the aforesaid operations are repeated to generate a new second template value to obtain a new updated template value. The storage unit (14) stores the first template value, each second template value and each updated template value. A method of controlling the breathable gas supply system (10) and a computer program product implementing the method are also provided.

## Description

### FIELD OF THE INVENTION

The present invention relates to a breathable gas supply system and a control method thereof, and more particularly to a breathable gas supply system and a control method thereof capable of varying a breath template in real time corresponding to the breathing condition of a patient. The present invention also relates to a computer program product implementing the method.

### BACKGROUND OF THE INVENTION

Some patients are incapable of breathing to obtain sufficient oxygen by themselves due to diseases or poor physical conditions and are in need of a breathable gas supply system to maintain respiration and sufficient body oxygen concentration. The breathable gas supply system can measure the apnea-hypopnea index (AHI) of a patient to prevent partial and complete obstruction of upper airway of the patient during respiration or sleep to avoid possible dangers caused therefrom.

In determining the AHI, normal respiration, i.e. a breath template, is generally used for comparison. For conventional breathable gas supply systems, a fixed or constant breath template is established after the system is switched on for use during the whole sleep process of the patient. However, there are several different stages in the sleep process, such as shallow sleep phase, deep sleep phase, rapid eye movement sleep phase, and awakening phase, and different stages are featured by different respiration patterns. Therefore, if a fixed or constant breath template is used as the basis for comparison during the whole sleep process, the real respiration conditions of the patient cannot be effectively evaluated and errors may exist in the determination. Accordingly, there is a need in the art to improve the way of breath template establishment in the conventional breathable gas supply systems.

### SUMMARY OF THE INVENTION

Provided herein is a breathable gas supply system capable of varying breath templates in real time corresponding to the respiration state of a patient. The dynamic breath templates may be used as the basis or reference for the determination of respiration states at different sleep stages.

Accordingly, disclosed herein is a breathable gas supply system for providing a breathable gas to a patient via a flow channel, the breathable gas supply system comprising a flow sensor, a control unit and a storage unit. The flow sensor is configured to detect a gas state of the flow channel. The control unit is electrically connected with the flow sensor, the control unit providing an initial template value and continuously receiving a sampled breath value from the flow sensor for repetitively performing a first breath sampling process; wherein when the first breath sampling process has been performed for a first preset number of times, the control unit generates a first template value for replacing the initial template value based on all first breath sampling processes; after the first template value has been generated, the control unit continuously receives the sampled breath value from the flow sensor for repetitively performing a second breath sampling process; when the second breath sampling process has been performed for a second preset number of times, the control unit generates a second template value based on all second breath sampling processes; the first template value and the second template value are then integrated to obtain an updated template value for replacing the first template value, and the above-cited operations are repeated to generate a new second template value so as to obtain a new updated template value. The storage unit is electrically connected with the control unit to store the first template value, each second template value and each updated template value.

Also disclosed is a method of controlling the aforesaid breathable gas supply system, comprising the following steps: (a) providing an initial template value and continuously receiving a sampled breath value through a flow channel for repetitively performing a first breath sampling process; (b) when the first breath sampling process has been performed for a first preset number of times, generating a first template value for replacing the initial template value based on all first breath sampling processes; (c) after the first template value has been generated, continuously receiving the sampled breath value through the flow channel for repetitively performing a second breath sampling process; (d) when the second breath sampling process has been performed for a second preset number of times, generating a second template value based on all second breath sampling processes; (e) integrating the first template value and the second template value to obtain an updated template value for replacing the first template value; and (f) repeating steps (c) to (e).

The computer program product disclosed herein is configured for detecting a gas state of a flow channel. When being loaded into the computer, the computer program product may perform the aforesaid control method of the breathable gas supply system.

In one embodiment, a breathable gas supply system for providing a breathable gas to a patient via a flow channel comprises: a flow sensor for detecting a gas state of the flow channel; a control unit providing an initial template value and continuously converting the gas state detected by the flow sensor into a sampled breathe value for repetitively performing a first breath sampling process, wherein when the first breath sampling process has been performed for a first preset number of times, the control unit generates a first template value for replacing the initial template value based on all first breath sampling processes, wherein after the first template value has been generated, the control unit continuously converts the gas state of the flow channel into the sampled breathe value for repetitively performing a second breath sampling process, wherein when the second breath sampling process has been performed for a second preset number of times, the control unit generates a second template value based on all second breath sampling processes, and wherein the first template value and the second template value are integrated to obtain an updated template value for replacing the first template value, and the above-cited operations are repeated to generate an updated template value for replacing a previous template value; and a storage unit for receiving and storing the initial template value, the first template value, the second template value and each updated template value.

According to one embodiment of the above-recited breathable gas supply system, the first breath sampling process comprises acquiring a first signal feature of the sampled breathe value within a preset time period, and wherein when the first breath sampling process has been performed for the first preset number of times, the control unit generates the first template value from the first signal feature.

According to one embodiment of the above-recited breathable gas supply system, the first signal feature comprises a voltage value, a current value, a power value, a waveform amplitude, an area under a waveform curve, a frequency, a phase angle or a combination thereof.

According to one embodiment of the above-recited breathable gas supply system, the second breath sampling process comprises acquiring a second signal feature of the sampled breathe value within a preset time period, and wherein when the second breath sampling process has been performed for the second preset number of times, the control unit generates the second template value from the second signal feature.

According to one embodiment of the above-recited breathable gas supply system, the second signal feature comprises a voltage value, a current value, a power value, a waveform amplitude, an area under a waveform curve, a frequency, a phase angle or a combination thereof.

### BRIEF DESCRIPTION OF THE DRAWINGS

A more complete understanding of the subject matter can be derived by referring to the detailed description and claims when considered in conjunction with the following figures, wherein like reference numbers refer to similar elements throughout the figures.
FIG. 1 illustrates the schematic block diagram of a breathable gas supply system of the present disclosure;
FIG. 2 illustrates the flowchart of a first example of the method of controlling a breathable gas supply system according to the present disclosure;
FIG. 3 illustrates the flowchart of a second example of the method of controlling a breathable gas supply system according to the present disclosure;
FIG. 4 illustrates the flowchart of a third example of the method of controlling a breathable gas supply system according to the present disclosure; and
FIG. 5 illustrates the flowchart of the pretreatment step of the third example of the method of controlling a breathable gas supply system according to the present disclosure.

### DETAILED DESCRIPTION OF THE INVENTION

Since various aspects and embodiments are merely exemplary and not limiting, after reading this specification, skilled artisans appreciate that other aspects and embodiments are possible without departing from the scope of the invention. Other features and benefits of any one or more of the embodiments will be apparent from the following detailed description and the claims.

The use of "a" or "an" is employed to describe elements and components described herein. This is done merely for convenience and to give a general sense of the scope of the invention. Accordingly, this description should be read to include one or at least one and the singular also includes the plural unless it is obvious that it is meant otherwise.

As used herein, the terms "first," "second," "third" and the like are used for distinguishing between or referring identical or similar elements or structures and not necessarily for describing a sequential or chronological order thereof. It should be understood that the terms so used are interchangeable under appropriate circumstances or configurations without affecting the implementation of the present disclosure.

Furthermore, as used herein, the terms "comprises," "comprising," "includes," "including," "has," "having" or any other variation thereof are intended to cover a non-exclusive inclusion. For example, a component, structure, article, or apparatus that comprises a list of elements is not necessarily limited to only those elements but may include other elements not expressly listed or inherent to such component, structure, article, or apparatus.

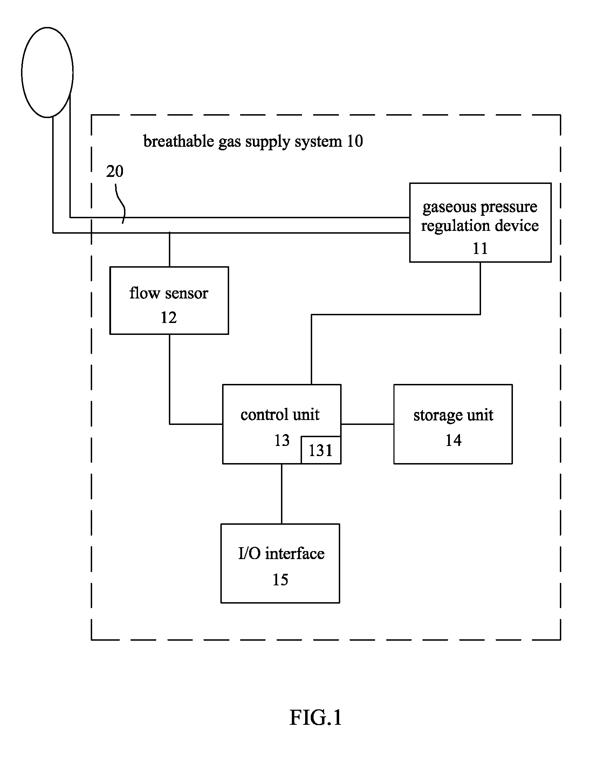

FIG. 1 illustrates the schematic block diagram of a breathable gas supply system 10 of the present disclosure. The breathable gas supply system 10 is arranged corresponding to a flow channel 20 for the respiration of a patient. A breathable gas is supplied to a patient through the flow channel 20, and the gas state in the flow channel 20 is detected to allow timely adjustment of a breath template. In this embodiment, the flow channel 20 is a gas delivery tube, which may comprise a gas delivery hose and an output part, such as a respiration mask or an exhaust pipe, wearable by the patient and other optional elements. The breathable gas may be air or oxygen.

In one embodiment, the breathable gas supply system 10 comprises a flow sensor 12, a control unit 13 and a storage unit 14. The control unit 13 is electrically connected with the flow sensor 12 and the storage unit 14. The flow sensor 12 is arranged in the flow channel 20 for detecting the gas state of the flow channel 20 and obtaining the sampled breath values of a patient. For example, a sensor unit may be arranged in the flow channel 20 for detecting the gas state, the sensor unit comprising a signal converter for converting the gas state into a sampled breath value which is then received by the control unit 13. The flow sensor 12 may be a flow rate sensor such that the gas state detected represents the gas flow rate, or the flow sensor 12 may also be a pressure sensor such that the gas state detected represents the gaseous pressure, depending on the need of use and design. Also, the flow sensor 12 may also comprise both a flow rate sensor and a pressure sensor. A flow rate sensor is used in the embodiments below as an example for the flow sensor 12 for illustration purpose, but this invention is not limited thereto.

The control unit 13 is used to provide an initial template value such that a preset breath template is available as soon as the system is booted. The control unit 13 may perform computation of new templates and replacement of old templates according to the data obtained and perform corresponding operations according to the input instruction from the user. The control unit 13 may be a central processing unit or a microprocessor or a combination of hardware and firmware or software such as operating systems or application programs. The initial template value may use a normal breath value corresponding to a body weight less than a predetermined value as the breath template value to be applicable to most patients. Generally, the predetermined body weight may be an average body weight of a population, 50 kg for example, such that the system uses the normal breath value of a person less than 50 kg as the initial template value; however, this invention is not limited thereto.

The storage unit 14 is used to receive and store the templates calculated and processed by the control unit 13, such that the templates required for computation of a new template are readily accessible for the control unit 13. The storage unit 14 may be a memory, a hard disk drive or an element with data storage capability.

The breathable gas supply system 10 further comprises a gaseous pressure regulation device 11 electrically connected with the control unit 13 and communicated with the flow channel 20. The gaseous pressure regulation device 11 adjusts the pressure of breathable gas in the flow channel 20 according to the instructions from the control unit 13. The gaseous pressure regulation device 11 may be a blower or other elements with air pressure regulation capability.

In addition, the breathable gas supply system 10 further comprises an input/output interface 15 electrically connected with the control unit 13 for receiving user's input and instruction and displaying the current state of the system and information associated thereto to serve as the communication medium between the user and the control unit 13. The input/output interface 15 may be a touch display panel, the combination of a display device and an interface program or the combination of a display device and push buttons.

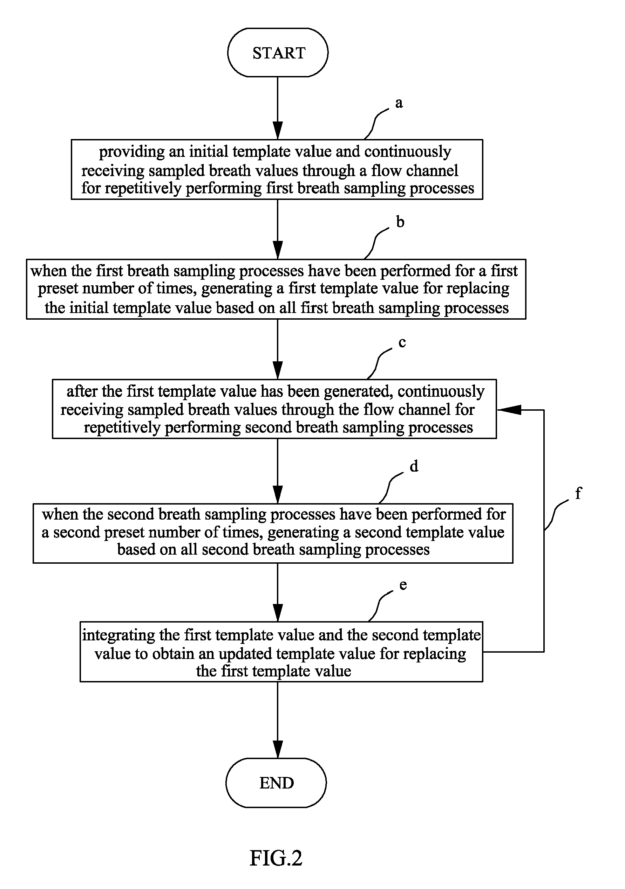

FIG. 2 illustrates the flowchart of a first example of the method of controlling a breathable gas supply system according to the present disclosure. It should be understood that although the breathable gas supply system 10 of FIG. 1 is used to illustrate the method of controlling a breathable gas supply system, the present invention is not limited thereto. As shown in FIG. 2, the method of controlling a breathable gas supply system comprises steps a to f, which are described in detail below.

Step a: providing an initial template value and continuously receiving sampled breath values through a flow channel 20 for repetitively performing first breath sampling processes

Before performing the method of controlling a breathable gas supply system, the output part of the flow channel 20 is mounted on the patient's mount/nose such that the flow channel 20 forms a closed gas circuit. After that, the flow sensor 12 arranged in the flow channel 20 begins detecting the gas state in the flow channel 20 to continuously sample the respiration process so as to obtain sampled breath values of the patient and transmit these values to the control unit 13. The control unit 13 will provide the initial template value as the breath template useful as soon as the system is booted. In addition, the control unit 13 begins continuously receiving the sampled breath values to repetitively perform the first breath sampling process.

In this embodiment, each first breath sampling process comprises obtaining a first breath waveform feature from each sampled breath value within a preset period of time. Each sampled breath value represents a breath waveform, and a corresponding waveform feature is obtained for each breath waveform. Therefore, in each first breath sampling process, the control unit 13 obtains a plurality of breath waveforms within the preset period of time and extracts first breath waveform features from these breath waveforms according to settings for subsequent operations. The first breath waveform feature herein may be any one or more of a maximum breath amplitude, a breath waveform area, an expiration amplitude, an inspiration amplitude, an expiration waveform area and an inspiration waveform area. In the following descriptions, the maximum breath amplitude of all breath waveforms within a preset period is used as an example of the first breath waveform feature, but this invention is not limited thereto, depending on the different needs or designs. The aforesaid preset period of time can be adjusted or changed to meet different needs; for example, the preset period may be tens of seconds or several minutes.

Step b: when the first breath sampling processes have been performed for a first preset number of times, generating a first template value for replacing the initial template value based on all first breath sampling processes

After the control unit 13 having repetitively performed the first breath sampling process for a first preset number of times, a plurality of first breath waveform features corresponding to the first present number of times may be obtained. Therefore, the control unit 13 may generate a first template value according to all obtained first breath waveform features. For example, the control unit 13 may aggregate all obtained maximum breath amplitudes and calculate the average amplitude to obtain the first template value and substitute the first template value for the initial template value to finish the preliminary breath template updating; however, the first template value can also be obtained by other ways. The first preset number of times can be adjusted according to different needs; for example, the first preset number of times may be several times or tens of times. The first template value may be saved in the storage unit 14.

In addition, in order to prevent errors in the first breath waveform features resulted from patient's coughing, sneezing or other accidental events during the repetitive first breath sampling processes, the control unit 13 may employ a filter module 131 to eliminate outliers from all first breath waveform features. For example, the control unit 13 may analyze and filter all obtained maximum breath amplitudes with the filter module 131. When the filter module 131 identifies one or more maximum breath amplitudes excessively greater or less than other maximum breath amplitudes, these outliers can be eliminated from subsequent calculation, and only maximum breath amplitudes passing the filter are taken in generating the first template value so as to reduce or inhibit errors caused by outliers in first template value generation.

Step c: after the first template value has been generated, continuously receiving sampled breath values through the flow channel 20 for repetitively performing second breath sampling processes

After the control unit 13 has generated the first template value, the flow sensor 12 incessantly detects the gas state in the flow channel 20 to continuously obtain sampled breath values of the patient and transmit them to the control unit 13. After the first template value has been generated, the control unit 13 begins repetitively performing the second breath sampling processes according to the continuously received sampled breath values.

In this embodiment, each second breath sampling process comprises obtaining second breath waveform features from a predetermined amount of the sampled breath values. In other words, in each second breath sampling process, the control unit 13 obtains a plurality of breath waveforms corresponding to the predetermined amount and extracts second breath waveform features from the breath waveforms according to settings for subsequent operations. Similarly, each second breath waveform feature may also be one or more of a maximum breath amplitude, a breath waveform area, an expiration amplitude, an inspiration amplitude, an expiration waveform area and an inspiration waveform area. In the following descriptions, the maximum breath amplitude of the predetermined amount of the breath waveforms is used as an example of the second breath waveform feature, but this invention is not limited thereto, depending on the different needs or designs. The aforesaid predetermined amount or number can be adjusted or changed to meet different needs; for example, the predetermined amount may be several to tens of sampled breath values.

To ensure the validity of the sampled breath values obtained, the control unit 13 may use the filter module 131 to eliminate any one of the predetermined amount of all sampled breath values which is less than a threshold value so as to obtain the second breath waveform features. For example, given that the sampled breath values represent breath waveforms, the threshold value may be set as 80% of the first template value. Therefore, the filter module 131 will eliminate any one with a breath waveform having a breath amplitude less than 80% of the first template value to avoid errors in subsequent calculations. However, the threshold value can be adjusted according to different needs.

Step d: when the second breath sampling processes have been performed for a second preset number of times, generating a second template value based on all second breath sampling processes

As in step b, after the control unit 13 having repetitively performed the second breath sampling processes for a second preset number of times, a plurality of second breath waveform features corresponding to the second present number of times can be obtained, such that the control unit 13 may generate the second template value according to all obtained second breath waveform features. For example, the control unit 13 may aggregate all obtained maximum breath amplitudes and calculate the average amplitude to obtain the second template value; however, the second template value can also be obtained by other ways. The second preset number of times can be adjusted according to different needs; for example, the second preset number of times may be several times or tens of times. The second template value may be saved in the storage unit 14.

Similarly, in order to prevent errors in the second breath waveform features during the repetitive second breath sampling processes, the control unit 13 may employ the filter module 131 to eliminate outliers from all second breath waveform features, and only those second breath waveform features passing the filter are taken in generating the second template value so as to reduce or inhibit errors caused by outliers in second template value generation.

Step e: integrating the first template value and the second template value to obtain an updated template value for replacing the first template value

After generating the second template value, the control unit 13 reads the first template value and the second template value from the storage unit 14 and integrates the two values to obtain the updated template value. If the difference between the second template value and the first template value is too large, such as the second template value far greater than the first template value, direct replacement of the first template value with the second template value may cause abrupt heavy load uncomfortable for the patient. Therefore, according to the present disclosure, both the first template value and the second template value are manipulated to obtain the updated template value. In one embodiment, the control unit 13 calculates the average of the first template value and the second template value to generate the updated template value, but this invention is not limited thereto; for example, the control unit 13 may also employ weighting to calculate the weighted average of the first template value and the second template value to generate the updated template value, or generate the updated template value according to a prespecified ratio between the first template value and the second template value. The updated template value thus obtained can be used to replace the first template value for subsequent breath template updating. The updated template value may also be stored in the storage unit 14.

Step f: repeating steps (c) to (e)

After obtaining the updated template value to replace the first template value, the control unit 13 repeats steps (c) to (e) to continuously generate a new second template value and read from the storage unit 14 the new second template value and the previously generated new first template value, which are integrated to generate the new updated template value for replacing the previous first template value, thereby repeating the iteration calculations. Accordingly, the method of controlling a breathable gas supply system according to the present disclosure may immediately update the breath template according to the patient's conditions to supply breathable gas in a more effective and accurate way.

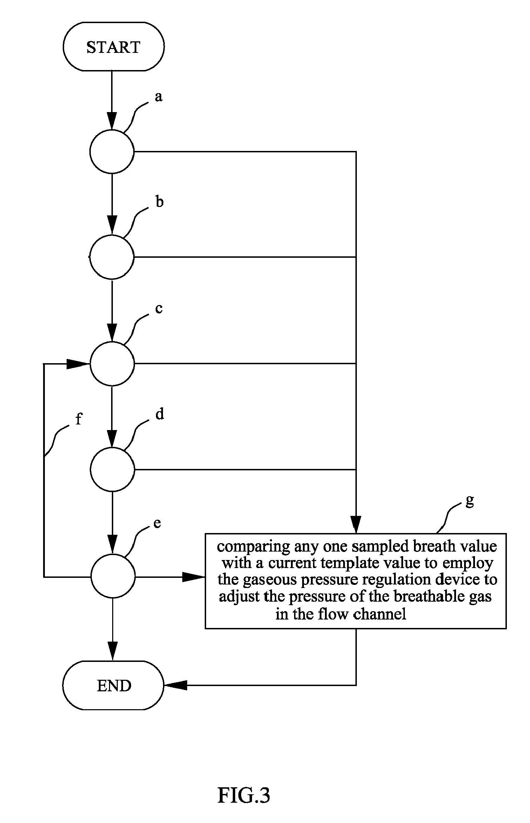

FIG. 3 illustrates the flowchart of a second example of the method of controlling a breathable gas supply system according to the present disclosure. In this embodiment, the method further comprises a step g: comparing any one sampled breath value with a current template value to notify the gaseous pressure regulation device 11 to adjust the pressure of the breathable gas in the flow channel 20, wherein the current template value is the initial template value, the first template value or the updated template value.

The breathable gas supply system of the present disclosure may be useful for determining an AHI of a patient, in which the control unit 13, by continuously comparing any sampled breath value obtained with the current template value during steps (a) to (e), determines whether to notify the gaseous pressure regulation device 11 to correspondingly adjust the pressure of the breathable gas in the flow channel 20. The breathable gas supply system 10 may employ a different breath template value at different stages correspondingly. The current template value may be any one of the initial template value, the first template value and the updated template value stored in the storage unit 14, and only one of these values is used as the current template value at a time point. In other words, before the first template value has been generated, the initial template value serves as the current template value; after the first template value has replaced the initial template value and before the second template value has be generated, the first template value serves as the current template value; after the updated template value has replaced the first template value, the updated template value serves as the current template value.

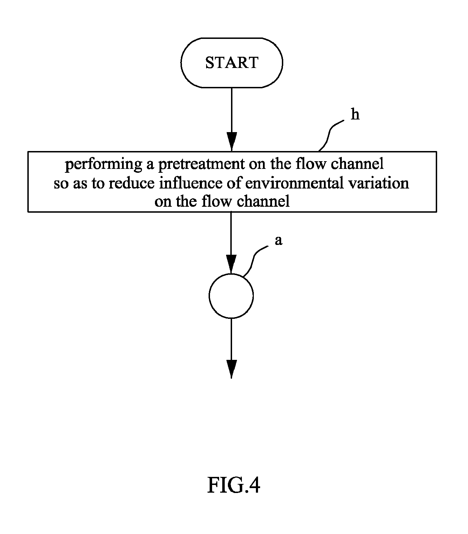

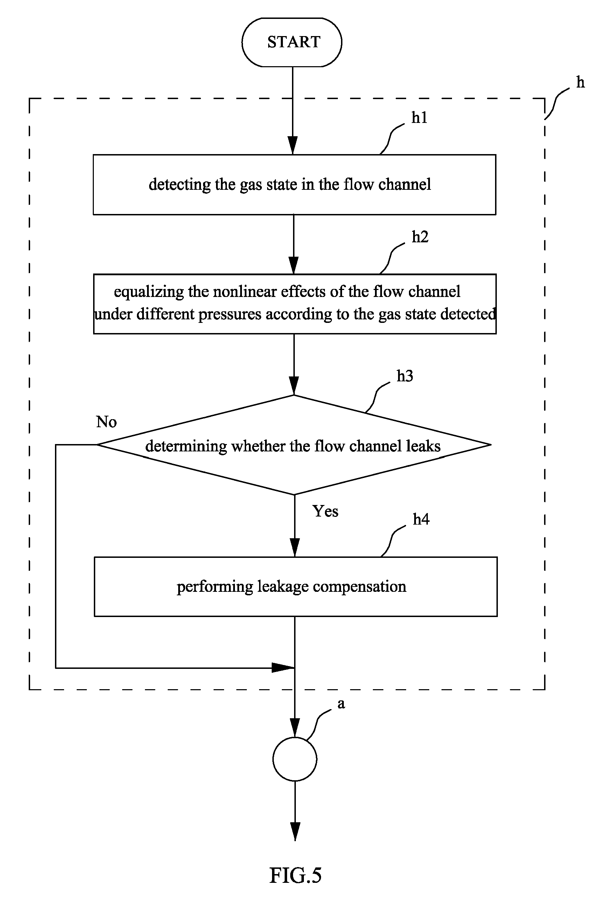

Refer to both FIGs. 4 and 5, wherein FIG. 4 illustrates the flowchart of a third example of the method of controlling a breathable gas supply system according to the present disclosure, and FIG. 5 illustrates the flowchart of the pretreatment step of the third example of the method of controlling a breathable gas supply system according to the present disclosure. As shown in FIG. 4, in this embodiment, the method of the present disclosure further comprises step h before step a.

Step h: performing a pretreatment on the flow channel 20 before performing the first breath sampling process so as to reduce influence of environmental variation on the flow channel 20

Before starting the breathable gas supply system 10 of the present disclosure, the condition or status of the flow channel 20 needs to be confirmed. Because the flow channel 20 is vulnerable to various environmental or external influences, such as leakage of the respiratory mask worn by the patient due to seams or separation and significant respiration change of the patient, errors may exist during the detection process of the flow sensor 12. Therefore, before the control unit 13 performs the first breath sampling process, a pretreatment process is preferably performed on the flow channel 20 so as to reduce the influence of environmental variation on the flow channel 20.

Said step h in this embodiment may comprise steps h1 through h4, which are described in detail below.

Step h1: detecting the gas state in the flow channel 20

At the beginning of the pretreatment process for the flow channel 20, various conditions such as gaseous pressure and flow rate variation of the flow channel 20 are measured to allow subsequent operations by the control module 13. Therefore, the breathable gas supply system 10 may employ the flow sensor 12 to detect the gas state in the flow channel 20 and transmit the result to the control module 13. In this embodiment, the flow sensor 12 may comprise both a flow rate sensor and a pressure sensor to respectively detect the flow rate and gaseous pressure in the flow channel 20.

Step h2: equalizing the nonlinear effects of the flow channel 20 under different pressures according to the gas state detected

According to the gas state detected, the control module 13 may determine the pressure difference in the flow channel 20 and use the gaseous pressure regulation device 11 to equalize of nonlinear effects of the flow channel 20 under different pressures, so as to minimize the influence of these effects on the flow channel 20.

Step h3: determining whether the flow channel 20 leaks

Similarly, the control module 13, according to the gas state measured such as pressure or flow rate, determines whether the flow channel 20 leaks; if it does, step h4 is performed; if not, the control module 13 employs the flow sensor 12 to perform breath sampling on the pretreated flow channel 20, followed by step a.

Step h4: performing leakage compensation if leaks exist

If the control module 13 determines that the current flow channel 20 leaks, the gaseous pressure regulation device 11 is used to perform leakage compensation on the flow channel 20 so as to minimize the influence of leakage on the flow channel 20. After compensation, the control module 13 employs the flow sensor 12 to perform breath sampling on the flow channel 20, followed by step a.

Accordingly, the breathable gas supply system 10 according to the present disclosure makes use of dynamic breath templates corresponding to different states of the patient, and the breath templates are adjusted in real time by iteration calculations, providing a design superior to conventional ones to reflect patient's real-time conditions more accurately.

The present disclosure also provides a computer program product configured for detecting a gas state of a flow channel. When being loaded into a computer, the computer program product may perform the aforesaid control method of the aforesaid breathable gas supply system.

In a second embodiment of the breathable gas supply system 10 according to the present disclosure, the control unit 13 of the breathable gas supply system 10 continuously acquires a gas state of the flow channel 20 detected by the flow sensor 12 and performs signal conversion on the gas state of the flow channel 20 to obtain a sampled breath value of a patient. The storage unit 14 receives and stores the initial template value and various template values processed and/or generated by the control unit 13, such that the control unit 13 can obtain any necessary template value to calculate a new template value.

When the second embodiment of the breathable gas supply system 10 is employed to implement the aforesaid control method, as Step a recited above, the control unit 13 similarly provides an initial template value to be received and stored in the storage unit 14 for subsequent use. The flow sensor 12 arranged in the flow channel 20 begins detecting the gas state of the flow channel 20, and the control unit 13, which is electrically connected with the flow sensor 12, performs signal conversion continuously on a gas state received to obtain a sampled breath value of the patient so as to perform the first breath sampling process repetitively.

In this embodiment, each first breath sampling process acquires a first signal feature from the sampled breath values within a preset time period. The first signal feature comprises a voltage value, a current value, a power value, a waveform amplitude, an area under a waveform curve, a frequency, a phase angle or a combination thereof. The preset time period may be adjusted according to different needs. In this embodiment, the sampled breath value is converted by the control unit 13, but it may also be converted by any converters for converting energy, form or pattern, such as rectifiers, A/D converters, transformers, filters, etc. In particular, the sampled breath value is the result of a gas state subjected to conversion at least once, and the control unit 13, based on the features of the sampled breath value, such as a voltage value, a current value, a power value, a waveform amplitude or the like recited above, generates a template value. In other words, no matter how many times the gas state has been converted, the sampled value is always in correspondence to or associated with the gas state detected by the flow sensor 12, and the control unit 13 calculates and generates a template value according to the features of the sampled breath value corresponding to the gas state.

As Step b recited above, when the first breath sampling processes have been performed for a first preset number of times, the control unit 13 generates a first template value for replacing the initial template value based on all first breath sampling processes, wherein the first preset number of times may be adjusted according to different needs.

As Step c recited above, after the first template value has been generated by the control unit 13, the control unit 13 continuously converts a gas state of the flow channel 20 into a sampled breath value for repetitively performing second breath sampling processes.

In this embodiment, each second breath sampling process acquires a second signal feature from a preset number of sampled breath values. The second signal feature comprises a voltage value, a current value, a power value, a waveform amplitude, an area under a waveform curve, a frequency or a phase angle. The preset number may be adjusted according to different needs.

As Step d recited above, when the second breath sampling processes have been performed by the control unit 13 for a second preset number of times, the control unit 13 generates a second template value based on all second breath sampling processes, wherein the second preset number of times may be adjusted according to different needs.

As Step e recited above, after a second template value has been generated, the control unit 13 integrates the first template value and the second template value to obtain an updated template value for replacing the first template value.

Finally, after the updated template value has been generated for replacing the first template value, the control unit 13 repeats step (c) to (e) to continuously calculate and generate an updated template value for replacing the previous template value.

The above detailed description is merely illustrative in nature and is not intended to limit the embodiments of the subject matter or the application and uses of such embodiments. Moreover, while at least one exemplary embodiment has been presented in the foregoing detailed description, it should be appreciated that a vast number of variations exist. It should also be appreciated that the exemplary one or more embodiments described herein are not intended to limit the scope, applicability, or configuration of the claimed subject matter in any way. Rather, the foregoing detailed description will provide those skilled in the art with a convenient guide for implementing the described one or more embodiments. Also, various changes can be made in the function and arrangement of elements without departing from the scope defined by the claims, which include known equivalents and foreseeable equivalents at the time of filing this patent application.

## Claims

1. A breathable gas supply system (10) for providing a breathable gas to a patient via a flow channel (20), the breathable gas supply system (10) comprising:
a flow sensor (12) for detecting a gas state of the flow channel (20);
a control unit (13) electrically connected with the flow sensor (12), **characterized by** the control unit (13) providing an initial template value and continuously receiving a sampled breath value from the flow sensor (12) for repetitively performing a first breath sampling process; wherein when the first breath sampling process has been performed for a first preset number of times, the control unit (13) generates a first template value for replacing the initial template value based on all first breath sampling processes; after the first template value has been generated, the control unit (13) continuously receives the sampled breath value from the flow sensor (12) for repetitively performing a second breath sampling process; when the second breath sampling process has been performed for a second preset number of times, the control unit (13) generates a second template value based on all second breath sampling processes; and the first template value and the second template value are integrated to obtain an updated template value for replacing the first template value, and the above-cited operations are repeated to generate a new second template value so as to obtain a new updated template value; and
a storage unit (14) electrically connected with the control unit (13) to store the first template value, each second template value and each updated template value.

2. The breathable gas supply system (10) of claim 1, wherein the first breath sampling process comprises obtaining a first breath waveform feature from all sampled breath values within a preset period, and wherein when the first breath sampling process has been performed for the first preset number of times, the control unit (13) generates the first template value according to all first breath waveform features.

3. The breathable gas supply system (10) of claim 2, wherein the second breath sampling process comprises obtaining a second breath waveform feature from a predetermined amount of the sampled breath values, and wherein when the second breath sampling process has been performed for the second preset number of times, the control unit (13) generates the second template value according to all second breath waveform features.

4. The breathable gas supply system (10) of claim 3, wherein the first breath waveform feature and the second breath waveform feature are individually selected from a maximum breath amplitude, a breath waveform area, an expiration amplitude, an inspiration amplitude, an expiration waveform area and an inspiration waveform area.

5. The breathable gas supply system (10) of claim 1, wherein the control unit (13) performs a pretreatment on the flow channel (20) before performing the first breath sampling process so as to reduce influence of environmental variation on the flow channel (20).

6. The breathable gas supply system (10) of claim 1, further comprising a gaseous pressure regulation device (11) electrically connected with the control unit (13), the control unit (13) comparing any one sampled breath value with a current template value to notify the gaseous pressure regulation device (11) to adjust the pressure of the breathable gas in the flow channel (20), wherein the current template value is the initial template value, the first template value or the updated template value.

7. The breathable gas supply system (10) of claim 1, wherein the updated template value is an average or a weighted average of the first template value and the second template value, and wherein the initial template value is less than a breath template value corresponding to a particular body weight.

8. The breathable gas supply system (10) of claim 3, wherein the control unit (13) comprises a filter module (131) for eliminating outliers from all first breath waveform features.

9. The breathable gas supply system (10) of claim 8, wherein the filter module (131) further eliminates any sampled breath value of the predetermined amount of the sampled breath values which is less than a threshold value to obtain the second breath waveform feature.

10. A method of controlling a breathable gas supply system (10) **characterized by** comprising the following steps:
(a) providing an initial template value and continuously receiving a sampled breath value through a flow channel (20) for repetitively performing a first breath sampling process;
(b) when the first breath sampling process has been performed for a first preset number of times, generating a first template value for replacing the initial template value based on all first breath sampling processes;
(c) after the first template value has been generated, continuously receiving the sampled breath value through the flow channel (20) for repetitively performing a second breath sampling process;
(d) when the second breath sampling process has been performed for a second preset number of times, generating a second template value based on all second breath sampling processes;
(e) integrating the first template value and the second template value to obtain an updated template value for replacing the first template value; and
(f) repeating steps (c) to (e).

11. The method of claim 10, wherein the first breath sampling process comprises obtaining a first breath waveform feature from all sampled breath values within a preset period, and wherein when the first breath sampling process has been performed for the first preset number of times, the first template value is generated according to all first breath waveform features.

12. The method of claim 11, wherein the second breath sampling process comprises obtaining a second breath waveform feature from a predetermined amount of the sampled breath values, and wherein when the second breath sampling process has been performed for the second preset number of times, the second template value is generated according to all second breath waveform features.

13. The method of claim 10, further comprising:
(g) comparing any one sampled breath value with a current template value to adjust the pressure of the breathable gas in the flow channel (20), wherein the current template value is the initial template value, the first template value or the updated template value.

14. The method of claim 10, wherein the initial template value, the first template value, the second template value and the updated template value are stored in a storage unit (14).

15. A computer program product for detecting a gas state of a flow channel (20), the computer program product, when being loaded into a computer, enabling implementation of the method of claim 10.
